# NEW EUROPEAN PATENT SPECIFICATION

(11) **EP 1 198 218 B2**
(45) Date of publication and mention of the opposition decision: **21.09.2016**
(45) Mention of the grant of the patent: 20.04.2005
(21) Application number: 01902015.5
(22) Date of filing: 11.01.2001
(51) Int. Cl.: A61K 8/06, A61K 8/20, A61K 8/29, A61Q 1/02, A61Q 19/08

(54) **OPTICAL MAKEUP COMPOSITION COMPRISING AN INTERFERENCE PIGMENT AND A METAL OXIDE PIGMENT**
OPTISCHE MAKEUP ZUSAMMENSETZUNG ENTHALTEND EIN INTERFERENZPIGMENT UND EIN METALLOXIDPIGMENT
COMPOSITION DE MAQUILLAGE OPTIQUE COMPRENANT UN PIGMENT D'INTEFERENCE ET UN PIGMENT D'OXYDE METALLIQUE

(30) Priority: 13.01.2000 US 482773
(43) Date of publication of application: 24.04.2002
(73) Proprietor: Color Access, Inc., Melville, New York 11747 (US)
(72) Inventor: DREHER, John, D., Sayville, NY 11782 (US); Stepniewski, George.J., Melville, New York 11747 (US)
(74) Representative: Hirsch & Associés
(86) International application number: PCT/US2001/001018
(87) International publication number: WO 2001/051017

(56) References cited:
- EP-A- 0 701 810
- EP-A2- 0 919 599
- WO-A-00/51551
- WO-A-96/03962
- WO-A-99/66883
- GB-A- 1 533 430
- US-A- 4 623 396
- Mearl: "Nacreous Pigments for Frosted and Iridescent Effects in Cosmetics, Cosmetic Formulary Pigment Properties", 1973
- New York Times article from 23 April 1996
- Mearl, "Flamenco Pearl and Color Pigments" brochure from 1983
- K. NISHIKATA ET AL.: 'A Natural-Looking Makeup' COSMETICS & TOILETRIES vol. 112, May 1997, pages 39 - 56

## Description

### Field of the Invention

The present invention relates to cosmetic compositions. In particular, the invention relates to compositions that can enhance the facial appearance by diminishing the viewer's ability to perceive fine lines and wrinkles on the face.

### Background of the invention

In today's highly youth-oriented culture, there is a tremendous emphasis on retaining a youthful appearance into middle age and beyond. The inevitable process of aging, whether chronological or UV-induced, strikes everyone, leaves the skin marked by a number of afflictions, the most noticeable of which start as fine lines and wrinkles, ultimately progressing to more discernable furrows and lines. The concern is not limited to the older person; even younger women now are more conscious of the early development of lines, which may result from excessive exposure to sun due to an active outdoor lifestyle. Although more affluent individuals can afford the luxury of periodic facelifts, the average person must find an alternate means for reducing the appearance of these flaws. In this regard, although makeup might seem to be an obvious choice to hide the problem, it often can accentuate it. In particular, the typical makeup components, such as metallic oxides, are intended primarily for coverage, and therefore confer an opacity to the composition, which, while excellent for evening out skin tone, may not be very flattering to the skin of older women, and further, in its tendency to accumulate in furrows, may actually serve to emphasize the deeper flaws rather than hiding them.

A recent advance in this area is the use of soft focus"- types of powders. These materials are spherical powders that are known in the cosmetic industry for their light scattering properties on the skin (see, for example, US: Patent No. 5,320,834). Such powders, for example, spherical sillicas, or polymethylmethacrylate, operate on the principle of diffusing light reaching the face in such a way that the overall appearance of the skin is somewhat blurred in the viewer's eye, thereby minimizing the viewer's opportunity to detect lines and wrinkles on tha skin. Although foundations containing these powders are quite effective and very attractive on the skin of older women, on younger skin they can confer somewhat of an opacity, so that the natural translucence of the young skin does not show through as effectively as would be desired.

There thus continues to be a need for a makeup that can reduce the appearance of lines and wrinkles on the skin, but at the same time will be lightweight, sheer, and translucent. Such a product will benefit both older and younger skins In the minimizing of surface flaws, yet at the same time, will permit the younger skin to retain its natural-looking glow. The present Invention now provides such a product.

### Summary of the Invention

The invention relates to a method of reducing the appearance of lines and wrinkles on the skin, which comprises applying to the skin a makeup composition comprising from about 1% to about 9% by weight based on the total weight of the composition of an interference pigment having a blue or violet reflectance, combined with at least one metal oxide pigment.

In preferred embodiments of the method of the invention:
- the interference pigment has a blue reflectance.
- the interference pigment has only a blue reflectance.
- the composition comprises titanium dioxide.
- the composition comprises titanium dioxide and iron oxide.
- the composition further comprises at least one inorganic, non-matte, non-spherical powder.
- the powder is selected from the group consisting of bismuth oxychloride, boron nitride, barium sulfate, mica, sericite, muscovite, synthetic mica, titanium oxide coated mica, titanium oxide coated bismuth oxychloride, titanium oxide coated talc, platelet iron oxides, aluminum powder, lauroyl lysine and platelet talc.
- the composition further comprises bismuth oxychloride.
- the composition comprises from about 2 to about 8% by weight of the interference pigment, based on the total weight of the composition.
- the metal is oxide present in an amount of about 0.1 to about 30% by weight, and the bismuth oxychloride is present in an amount of about 2 to about 10% by weight, based on the total weight of the composition.

The invention also relates to a skin-colored makeup composition comprising an interference pigment having a blue or violet reflectance in an amount of from about 1 to about 9% by weight, combined with at least one metal oxide pigment and an inorganic powder in an amount of about 2 to about 15% by weight, based on the total weight of the composition which comprises an interference pigment having only a blue reflectance, titanium dioxide and at least one iron oxide, and an inorganic non-matte, non-spherical powder selected from the group consisting of bismuth oxychloride, boron nitride, barium sulfate, mica, sericite, muscovite, synthetic mica, titanium oxide coated mica, titanium oxide coated bismuth oxychloride, titanium oxide coated talc, platelet iron oxides, aluminum powder, lauroyl lysine and platelet talc and in which the metal oxides are present in an amount of 0.1 to 30%.

In preferred embodiments of the make-up composition of the invention:
- the powder is bismuth oxychloride.
- the interference pigment is present in amount of about 2 to about 8% by weight, based on the total weight of the composition.

### Detailed Description of the Invention

It has been unexpectedly discovered that the presence of an interference pigment having a blue or violet reflectance in a standard makeup composition, particularly a foundation, can create the illusion of substantially flawless skin, by "deceiving" the observer's eyes into not perceiving the lines and wrinkles that are actually present on the wearer's skin. The human eye has different sensitivities to different wavelengths of light, and the blue-violet wavelengths are ones to which the eye is least sensitive. Surprisingly, the presence of corresponding colors of interference pigment has this effect on the eye when used in a makeup, In that when the makeup is applied to the face and receives light, it reflects that fight back in such a way that the viewer does not see the wrinkles lying beneath it, but rather sees a smooth, unlined complexion. Interference pigments are defined as thin platelike layered particles having a high refractive Index, which, at a certain thickness, produce interference colors, resulting from the interference of typically two, but occasionally more, light reflections, from different layers of the plate. The most common examples of interference pigments are micas layered with 50-500nm films of TiO₂, Fe₂O₃, or Cr₂O₃, or combinations thereof. The interference pigment of the present invention produces a blue or violet color, at wavelength of about 380-490 nm, from the interference layer. The mica base may be colored or uncolored. Such pigments are not new, and have been previously used in cosmetics, primarily in very small quantities as a colorant in skin care products to confer a pearlescence to the product, or at high levels in makeup products such as eyeshadows, lipsticks or blushes, to confer a blue pearlescent color. They have also been previously used in makeup products, at relatively high levels, i.e., 10% or more, to disguise the appearance of major skin imperfections, such as hyperchromic pigmentation on the face, for example, port wine stains or hemangiomas. In the latter usage, unlike the present, the makeup composition containing the pigment is intended to complement the hyperchromic pigmentation, rather than match the normal color of the skin surfaceto which it is applied. Therefore, the previous known uses of these blue or violet pigments have neither been recognized nor exploited this unique property.

The blue or violet pigment is employed in the composition in an amount of about 1 to about 9%, preferably about 4 to about 8% by weight of the total composition. The blue or violet interference pigments of the invention are available commercially from a number of sources. The preferred blue or violet interference pigment is a titanated mica which is available, for example, from Rona under the tradename Timiron®, or from Engelhard under the tradename Flamenco®. The latter pigments have only a blue or violet reflectance color. However, the interference pigment used may also be one having not only a blue or violet reflectance, but also one or more other reflectance colors, by virtue of the presence of one or more additional interference layers, that may or may not be the traditional types of substrates. Examples of such interference pigments are available commercially from BASF under the tradename Sicopearl®, the latter containing interference layers comprising silica, iron oxide, and optionally, aluminum. Additional such pigments are also available from Flex Products, Inc., under the tradename Chromaflair®.

In the makeup compositions of the invention, the blue or violet interference pigment is combined with at least one metal oxide pigment of the type ordinarily used in color cosmetics, to give a "skin-colored" appearance to the formula. Examples of useful pigments include iron oxides (yellow; red, brown or black), titanium dioxide(white), zinc oxide, chrome oxide(green), chrome hydrate(green), ultramarines, manganese violet, ferric ferrocyanide, carmine 40, ferric ammonium ferrocyanide, or combinations thereof. Particularly preferred is a combination of one or more iron oxides with titanium dioxide. These pigments are typically present in an amount of about .1 to about 30%, preferably about 0,1 to about 20%.

Organic pigments may also optionally be included; these include natural colorants and synthetic monomeric and polymeric colorants. Exemplary are phthalocyanine blue and green pigment, diarylide yellow and orange pigments, and azo-type red and yellow pigments such as toluidine red, litho red, naphthol red and brown pigments. Also useful are lakes, which are pigments formed by the precipitation and absorption of organic dyes on an insoluble base, such as alumina, barium, or calcium hydrates. Particularly preferred lakes are primary FD&C or D&C Lakes and blends thereof. Stains, such as bromo dyes and fluorescein dyes can also be employed.

The composition also contains an inorganic powder. It has been observed that, with the use of interference pigments producing only a blue or violet reflectance color in combination with metal oxides alone, these compositions do produce the desired reduction in appearance of fine lines and wrinkles, but it is an "all-or-nothing" appearance: the viewer perceives the full benefit of the reflectance from the interference pigment when looking at the skin from the specular angle, or head-on; however, when the same skin is viewed at an incident angle, the reflectance from the interference layer is not visible, and only the pigment is seen. Thus, the transition between these two views is quite sharp, and therefore somewhat less than ideal. However, it has been unexpectedly discovered that the transition between viewing at specular avid incident angles can be softened by the inclusion in the formula of an inorganic powders, such as a silica or polymethylmethacrylate. The powder is a non-matte powders, in an amount of about 2 to about 15%. The most preferred powders for this purpose are platelike, non-spherical powders that confer some luster, but not an overt shine, so that there is still some reflectance, albeit muted, even when it is not coming directly from the interference pigment. To achieve the maximum benefit of this effect, the powder is preferably uncolored and has an average particle size that is relatively small, about 2 to 50µ, more preferably about 3- 20µ, most preferably about 3 to 6µ. Such powders are selected from: bismuth oxychloride, boron nitride, barium sulfate, mica, sericite, muscovite, synthetic mica, titanium oxide coated mica, titanium oxide coated bismuth oxychloride, titanium oxide coated talc, platelet iron oxides, metal powders such as aluminum, lauroyl lysine and platelet talc. The composition containing these powders confers a more uniform appearance to the skin, providing a greater clarity and depth, with a soft, translucent glowing effect characteristic of young, healthy skin, than does the same composition without the powder. In a particularly preferred embodiment, the powder used is bismuth oxychloride.

The makeup compositions of the invention can take any form that is normally used for foundations. For example, the composition can be aqueous or anhydrous, and can be a gel, a water-in-oil emulsion, an oil-in-water emulsion, a stick, a solid, and any other appropriate form. The preferred form, however, is an oil-in-water emulsion, and particularly a silicone-in-water emulsion. The makeup compositions of the invention can also include a variety of optional cosmetic ingredients, such as thickeners, emulsifiers, preservatives, antioxidants, sunscreens, emollients, surfactants, and the like.

The compositions of the invention are used in the same manner as any typical foundation, i.e., the user applies the formulation to the skin on which the lines to be disguised appear, with, for example, the fingers or an appropriate applicator.

The invention will be further Illustrated by the following non-limiting examples:

### Example 1. This example Illustrates a formula for an oil-in-water emulsion of the Invention.

| Material | Weight percent |
|---|---|
| Phase I | |
| Purified water | QS |
| Triethanolamine | 1.00 |
| Methyl paraben | 0.35 |
| Phase II | |
| Steareth-21 | 0.50 |
| Phase III | |
| Titanium dioxide | 4.50 |
| Red iron oxide | 0.50 |
| Yellow Iron oxide | 1.80 |
| Black iron oxide | 0.10 |
| Phase IV | |
| Purified water | 4.00 |
| Phase V | |
| Butylene glycol | 4.00 |
| Phase VI | |
| Magnesium aluminum silicate | 0.50 |
| Phase VII | |
| Blue titaneted mica | 4.00 |
| Bismuth oxychloride | 4.00 |
| Phase VIII | |
| Dimethicone | 15.00 |
| Stearic acid | 1.85 |
| Steareth-2 | 0.30 |
| Propyl paraben | 0.10 |
| Ethyl paraben | 0.15 |
| Glyceryl dilaurate | 1.50 |
| Polydecene | 2.00 |
| Phenoxyethanol | 0.50 |

Phase I is mixed and heated to 45°C under propeller agitation. Phase II is added and mixed until uniform. Phase III Is sprinkled In and mixing Is continued. Phases I-III are the milled in a suitable milling machine (i.e.: colloid mill, ball mill, etc.) Milling Is complete when no pigment particles are visible when the mixture Is pressed between 2 glass slides. Phase IV Is used to rinse out the mill. Phases V, VI, and VII are added to phases I-II under propeller type mixing, and until uniform. Phases I-VII are known as the "water phase". This is then heated to 75°C.

Phase VIII is heated in a suitable container to 80°C under similar propeller mixing. This Is the "oil phase".

When both phases are at temperature, the oil phase Is slowly added to the water phase. A drop in type-homogenizer is then inserted and the speed is set so as not to Introduce air into the batch. The temperature Is held between 75 and 80°C for 15 minutes then the batch Is cooled to 25°C via propeller mixing.

### Example 2. This example Illustrates a formulation which Is a water-in-oil emulsion of the present invention.

| Material | Weight percent |
|---|---|
| Phase I | |
| Sorbitan sesquioleate | 1.50 |
| Dimethicone | 6.00 |
| phrase II | |
| Titanium dioxide | 3.50 |
| Red Iron oxide | 0.60 |
| Yellow iron oxide | 1.20 |
| Black Iron oxide | 0.10 |
| Phase III | |
| Blue titanated mica | 4.00 |
| Bismuth oxychloride | 4.00 |
| Phase IV | |
| Cyclomethicone/dimethicone copolyol | 15.00 |
| Cyclomethicone | 4.00 |
| Octyldodecanol | 2.00 |
| Isononyl Isononanoate | 2.00 |
| BHT | 0.05 |
| Propylparaben | 0.10 |
| Phase V | |
| Phenyl trimethicone | 12.00 |
| Phase VI | |
| Tribehenin | 1.00 |
| Phase VII | |
| Purified water | 39.05 |
| Imidazoildinyl urea | 0.15 |
| Phase VIII | |
| Butylene glycol | 2.00 |
| Laureth-7 | 0.25 |
| Magnesium sulfate | 1.50 |

Phase I is mixed under propeller type mixing In a suitable container until uniform. Phase II is sprinkled In and mixed until uniform. Phases I-II are the milled In a suitable milling machine (i.e.: colloid mill, ball mill, etc.) Milling Is complete when no pigment particles are visible when the mixture is pressed between 2 glass slides. When complete, phase III Is sprinkled Into the combined phases I and II. Phase IV is then added to phases I-III under propeller agitation.

Phase V and VI are combined in a suitable container and heated to 70°C under propeller mixing until uniform. Phases I-IV are placed In a suitable container and heated to 50°C. Phase V and VI is then added to combined phases I-IV. The temperature Is maintained between 50 and 55°C under propeller mixing.

Phases VII and VIII are combined In a suitable container and heated to 50°C under propeller mixing until uniform.

When at temperature, Phases VII and VIII are slowly added to phases I-VI under combined propeller agitation. A drop in-type homogenizer is then inserted and the speed Is set so as not to Introduce air into the batch. The temperature is held between 50 and 55°C for 15 minutes, then the batch is cooled to 25°C via propeller mixing..

## Claims

1. A method of reducing the appearance of lines and wrinkles on the skin, which comprises applying to the skin a makcup composition comprising from about 1 to about 9% by weight, based on the total weight of the composition, of an interference pigment having a blue or violet reflectance, combined with at least one metal oxide pigment.

2. The method of claim 1 in which the interference pigment has a blue reflectance.

3. The method of claim 1 in which the interference pigment has only a blue reflectance.

4. The method of any of claims 1 to 3 in which the composition comprises titanium dioxide.

5. The method of any of claims 1 to 4 in which the composition comprises titanium dioxide and iron oxide.

6. The method of any of claims 1 to 5 in which the composition further comprises at least one inorganic, non-matte, non-spherical powder.

7. The method of claim 6 in which the powder is selected from the group consisting of bismuth oxychloride, boron nitride, barium sulfate, mica, sericite, muscovite, synthetic mica, titanium oxide coated mica, titanium oxide coated bismuth oxychloride, titanium oxide coated talc, platelet iron oxides, aluminum powder, lauroyl lysine and platelet talc.

8. The method of claim 7 in which the composition further comprises bismuth oxychloride.

9. The method of any one of claims 1 to 8 in which the composition comprises from about 2 to about 8% by weight of the interference pigment, based on the total weight of the composition.

10. The method of claim 9 in which the metal is oxide present in an amount of about 0.1 to about 30% by weight, and the bismuth oxychloride is present in an amount of about 2 to about 10% by weight, based on the total weight of the composition.

11. A skin-colored makeup composition comprising an interference pigment having a blue or violet reflectance in an amount of from about 1 to about 9% by weight, combined with at least one metal oxide pigment and an inorganic powder in an amount of about 2 to about 15% by weight, based on the total weight of the composition, which comprises an interference pigment having only a blue reflectance, titanium dioxide and at least one iron oxide; and an inorganic non-matte, non-spherical powder selected from the group consisting of bismuth oxychloride, boron nitride, barium sulfate, mica, scricite, muscovite, synthetic mica, titanium oxide coated mica, titanium oxide coated bismuth oxychloride, titanium oxide coated talc, platelet iron oxides, aluminum powder, lauroyl lysine and platelet talc; and in which the metal oxides are present in an amount of 0.1 to 30%.

12. The composition of claim 11 in which the powder is bismuth oxychloride.

13. The composition of any of claims 11 to 12 in which the interference pigment is present in amount of about 2 to about 8% by weight, based on the total weight of the composition.

## Patentansprüche

1. Ein Verfahren zur Reduktion des Sichtbarwerdens von Linien und Falten auf der Haut, welches das Auftragen einer Makeup-Mischung, welche von ungefähr 1 bis ungefähr 9 Gew.%, basierend auf dem Gesamtgewicht der Mischung, ein Interferenzpigment mit blauem oder violettem Rückstrahlungsvermögen, kombiniert mit mindestens einem Metalloxidpigment beinhaltet, auf die Haut.

2. Das Verfahren nach Anspruch 1, in dem das Interferenzpigment ein blaues Rückstrahlungsvermögen hat.

3. Das Verfahren nach Anspruch 1, in dem das Interferenzpigment nur ein blaues Rückstrahlungsvermögen hat.

4. Das Verfahren nach einem der Ansprüche 1 bis 3, in dem die Mischung Titandioxid beinhaltet.

5. Das Verfahren nach einem der Ansprüche 1 bis 4, in dem die Mischung Titandioxid und Eisenoxid beinhaltet.

6. Das Verfahren nach einem der Ansprüche 1 bis 5, in dem die Mischung ferner mindestens einen anorganischen, nicht matten, nicht-kugelförmigen Puder beinhaltet.

7. Das Verfahren nach Anspruch 6, in dem der Puder aus der Gruppe, die aus Wismuthypochlorid, Bornitrid, Bariumsulfat, Glimmer, Sericit, Muscovit, synthetischem Glimmer, Titanoxid-beschichtetem Glimmer, Titanoxid-beschichtetem Wismuthypochlorid, Titanoxid-beschichtetem Talkum, Eisenoxidplättchen, Aluminiumpulver, Lauroyllysin und Talkumplättchen besteht, ausgewählt wird.

8. Das Verfahren nach Anspruch 7, in dem die Mischung ferner Wismuthypochlorid beinhaltet.

9. Das Verfahren nach einem der Ansprüche 1 bis 8, in dem die Mischung von ungefähr 2 bis ungefähr 8 Gew.%, basierend auf dem Gesamtgewicht der Mischung, Interferenzpigment beinhaltet.

10. Das Verfahren nach Anspruch 9, in dem das Metalloxid in einer Menge von ungefähr 0,1 bis ungefähr 30 Gew.% und das Wismuthypochlorid in einer Menge von ungefähr 2 bis ungefähr 10 Gew.%, basierend auf dem Gesamtgewicht der Mischung, vorhanden ist.

11. Eine hautfarbene Makeup-Mischung, welche ein Interferenzpigment mit blauem oder violettem Rückstrahlungsvermögen in einer Menge von ungefähr 1 bis ungefähr 9 Gew.%, kombiniert mit mindestens einem Metalloxidpigment und einem anorganischen Puder in einer Menge von ungefähr 2 bis ungefähr 15 Gew.%, basierend auf dem Gesamtgewicht der Mischung, beinhaltet, die ein Interferenzpigment mit nur ein blaues Rückstrahlungsvermögen, und ein nichtkugelförmiger, nicht matter anorganische Puder, aus der Gruppe bestehend aus Wismuthypochlorid, Bornitrid, Bariumsulfat, Glimmer, Sericit, Muscovit, synthetischem Glimmer, Titanoxid-beschichtetem Glimmer, Titanoxid-beschichtetem Wismuthypochlorid, Titanoxid-beschichtetem Talkum, Eisenoxidplättchen, Aluminiumpulver, Lauroyllysin und Talkumplättchen besteht, ausgewählt wird, in dem die Metalloxide in einer Menge von ungefähr 0,1 bis ungefähr 30 Gew.% enthalten sind.

12. Die Mischung nach Anspruch 11, in dem der Puder Wismuthypochlorid ist.

13. Die Mischung nach einem der Ansprüche 11 bis 12, in welcher das Interferenzpigment in einer Menge von ungefähr 2 bis ungefähr 8 Gew.%, basierend auf dem Gesamtgewicht der Mischung,

## Revendications

1. Procédé pour réduire l'apparition de stries et de rides sur la peau, qui comprend l'application sur la peau d'une composition de maquillage comprenant d'environ 1 à environ 9% en poids, exprimés par rapport au poids total de la composition, d'un pigment d'interférence, ayant un facteur de réflexion pour le bleu ou le violet, combiné avec au moins un pigment à base d'oxyde métallique.

2. Procédé selon la revendication 1, dans lequel le pigment d'interférence a un facteur de réflexion pour le bleu.

3. Procédé selon la revendication 1, dans lequel le pigment d'interférence n'a qu'un facteur de réflexion pour le bleu.

4. Procédé selon l'une quelconque des revendications 1 à 3, dans lequel les compositions comprennent du dioxyde de titane.

5. Procédé selon l'une quelconque des revendications 1 à 4, dans lequel la composition comprend du dioxyde de titane et du dioxyde de fer.

6. Procédé selon l'une quelconque des revendications 1 à 5, dans lequel la composition comprend en outre au moins une poudre inorganique, non mate, non sphérique.

7. Procédé selon la revendication 6, dans lequel la poudre est choisie dans le groupe constitué par l'oxychlorure de bismuth, le nitrure de bore, le sulfate de baryum, le mica, la séricite, la muscovite, le mica synthétique, le mica revêtu d'oxyde de titane, l'oxychlorure de bismuth revêtu d'oxyde de titane, le talc revêtu d'oxyde de titane, les oxydes de fer lamellaires, la poudre d'aluminium, la lauroyllysine et le talc lamellaire.

8. Procédé selon la revendication 7, dans lequel la composition comprend en outre de l'oxychlorure de bismuth.

9. Procédé selon l'une quelconque des revendications 1 à 8 dans lequel la composition comprend d'environ 2 à environ 8% en poids, exprimés par rapport au poids total de la composition, du pigment d'interférence.

10. Procédé selon la revendication 9, dans lequel, exprimée par rapport au poids total de la composition, le métal est un oxyde présent en une quantité d'environ 0,1 à environ 30% en poids, et l'oxychlorure de bismuth est présent en une quantité d'environ 2 à environ 10% en poids.

11. Composition de maquillage de couleur peau comprenant un pigment d'interférence ayant un facteur de réflexion pour le bleu ou le violet en une quantité, d'environ 1 à environ 9% en poids, combiné avec au moins un pigment d'oxyde métallique et une poudre inorganique en une quantité d'environ 2 à environ 15% en poids, quantités exprimées par rapport au poids total de la composition, qui comprend un pigment d'interférence n'ayant qu'un facteur de réflexion pour le bleu, du dioxyde de titane et au moins un oxyde de fer et une poudre inorganique non sphérique, non mate choisie dans le groupe constitué par l'oxychlorure de bismuth, le nitrure de bore, le sulfate de baryum, le mica, la séricite, la muscovite, le mica synthétique, le mica revêtu d'oxyde de titane, l'oxychlorure de bismuth revêtu d'oxyde de titane, le talc revêtu d'oxyde de titane, des oxydes de fer lamellaires, la poudre d'aluminium, la lauroyllysine et le talc lamellaire, et dans lequel les oxydes métalliques sont présents en une quantité d'environ 0,1 à environ 30% en poids.

12. Composition selon la revendication 11, dans laquelle la poudre est de l'oxychlorure de bismuth.

13. Composition selon l'une quelconque des revendications 11 à 12, dans laquelle le pigment d'interférence est présent en une quantité, exprimée par rapport au poids total de la composition, d'environ 2 à environ 8% en poids.
